(19) Europäisches Patentamt
European Patent Office
Office européen des brevets

(11)  **EP 2 045 648 B1**

(12)  **EUROPEAN PATENT SPECIFICATION**

(45) Date of publication and mention
of the grant of the patent:
**11.04.2012  Bulletin 2012/15**

(51) Int Cl.:
*G02C 7/04* (2006.01)        *A61F 2/16* (2006.01)

(21) Application number: **08165665.4**

(22) Date of filing: **01.10.2008**

(54) **Zonal diffractive multifocal intraocular lenses**

Zonale, diffraktive, multifokale Intraokularlinsen

Lentilles intraoculaires multifocale à diffraction zonale

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **02.10.2007  US 997279 P**

(43) Date of publication of application:
**08.04.2009  Bulletin 2009/15**

(60) Divisional application:
**11181646.8 / 2 400 339**
**11185333.9 / 2 407 816**

(73) Proprietor: **Novartis AG**
**4056 Basel (CH)**

(72) Inventors:
• **Simpson, Michael J.**
**Arlington, TX 76012 (US)**
• **Karakelle, Mutlu**
**Fort Worth, TX 76132 (US)**

(74) Representative: **Hanna, Peter William Derek et al**
**Hanna Moore & Curley**
**13 Lower Lad Lane**
**Dublin 2 (IE)**

(56) References cited:
**EP-A- 0 457 553        WO-A-91/09336**
**WO-A-2006/060480      DE-U1-202005 009 124**
**US-A- 5 056 908        US-B1- 7 073 906**

**Description**

**BACKGROUND**

The present invention relates generally to multifocal ophthalmic lenses, and more particularly, to multifocal intraocular lenses that can provide refractive and diffractive optical focusing powers.

Intraocular lenses are routinely implanted in patients' eyes during cataract surgery to replace a clouded natural crystalline lens. Some IOLs employ diffractive structures to provide a patient with not only a far-focus power but also a near-focus power. In other words, such IOLs provide the patient with a degree of accommodation (sometimes referred to as "pseudoaccommodation"). Although patients having such IOLs generally enjoy the versatile focusing properties of these lenses, a small percentage make observations about the clarity of their far vision, especially in photoopic conditions.

Accordingly, there is still a need for enhanced multifocal ophthalmic and, in particular, multifocal intraocular lenses.

**SUMMARY**

In one aspect, the present invention provides an intraocular lens (IOL), in accordance with claims which follow, which comprises an optic having an anterior surface and a posterior surface, where the optic includes a central refractive region for providing one refractive focusing power. A diffractive region is disposed on at least one of the lens surfaces for providing a near and far diffractive focusing power. In many cases, the refractive and diffractive far focusing powers are substantially equal.

In a related aspect, in the above IOL, the diffractive region, is surrounded by an outer refractive region. In some cases, the central refractive region can have a diameter in a range of about 0.5 mm to about 2 mm.

In another aspect, the diffractive region includes a plurality of diffractive zones (e.g., 2 to 20 zones) that are separated from one another by a plurality of steps. According to a comparative example not covered by the claims the steps exhibit substantially uniform heights, in others their heights can be non-uniform. For example, the steps can be apodized such that their heights decrease as a function of increasing radial distance from a center of the optic. According to claim 1, the apodized steps exhibit increasing heights as a function of increasing radial distance from the center of the optic - that is the steps can be "reverse apodized." In another case, the step heights can increase from an inner radial boundary of the diffractive region to an intermediate location in that region followed by a decrease to the region's outer radial boundary, and vice versa.

According to a comparative example not covered by the claims, in the above ophthalmic lens, the central and the outer refractive regions provide different refractive powers, e.g., the central region can provide a far-focusing power and the outer refractive region can provide a near-focusing power or *vice versa.* The diffractive region can, in turn, provide diffractive near and far focusing powers corresponding to the refractive near and far focusing powers provided by the central and the outer regions.

According to a comparative example not covered by the claims, a multifocal ophthalmic lens is disclosed that includes an optic having a central refractive region for providing a near-focus optical power and a diffractive region for providing a diffractive far-focus and a diffractive near-focus optical power. In many cases, the refractive and the diffractive near-focus powers are substantially equal.

According to a comparative example not covered by the claims, the invention provides an ophthalmic lens (e.g., an IOL) that includes an optic having a central refractive region for providing a refractive far-focus optical power, and a diffractive region disposed on at least one surface of the optic for providing a diffractive near-focus and a diffractive intermediate-focus optical power. In some cases, the optic can also include an outer refractive region that contributes refractively to the lens's far-focus or near-focus power.

Further understanding of various aspects of the invention can be obtained by reference to the following detailed description in conjunction with the associated drawings, which are discussed briefly below.

The state of the art is represented by US-A-7, 073, 906.

**BRIEF DESCRIPTION OF THE DRAWINGS**

FIGURE 1A is a schematic top view of a multifocal IOL according to a comparative example not covered by the claims,

FIGURE 1B is a schematic side view of the multifocal IOL shown in FIGURE 1A,

FIGURE 2 shows a radial profile of the anterior surface of the IOL shown in FIGURES 1A and 1B from which the base profile of the anterior surface has been subtracted,

FIGURE 3 depicts graphs corresponding to local and integrated distributions of light energy between the far and the near focus of a hypothetical implementation of the IOL shown in FIGURES 1A and 1B for pupil sizes in a range of 0 to about 6 mm,

FIGURE 4A is a schematic side view of a multifocal IOL in accordance with one embodiment having a reverse-apodized diffractive region,

FIGURE 4B is a radial profile of the anterior surface (minus the base profile of the surface) of the IOL shown in FIGURE 4A,

FIGURE 5A is a schematic side view of a multifocal IOL according to an embodiment of the invention,

FIGURE 5B is a radial profile of the anterior surface (minus the surface base profile) of the IOL of FIGURE 5A, indicating that the steps separating different diffractive zones of a diffractive region disposed on the surface exhibit an increase in heights followed by a decrease as a function of increasing radial distance from the lens center,

FIGURE 5C is a radial profile of a surface (minus the surface base profile) of an IOL according to an embodiment in which the steps separating different diffractive zones of a diffractive region disposed on the surface exhibit a decrease in heights followed by an increase as a function of increasing radial distance from the lens center,

FIGURE 6 depicts graphs corresponding to local and integrated distributions of light energy between near and far foci of a lens having a diffractive region similar to that shown in FIGURE 5B,

FIGURE 7 is a radial profile of a surface (minus the surface base profile) of an IOL according to a comparative example not covered by the claims in which the steps separating different diffractive zones of a diffractive region disposed on the surface exhibit substantially uniform heights,

FIGURE 8 is a schematic side view of an IOL according to a comparative example not covered by the claims of the invention in which a diffractive region disposed on the lens's anterior surface extends to the periphery of the lens,

FIGURE 9 is a schematic side view of an IOL according to a comparative example not covered by the claims in which the lens's central refractive region provides a near-focus optical power,

FIGURE 10 is a schematic side view of an IOL according to a comparative example not covered by the claims in which the lens's central refractive region provides a far-focus optical power and a diffractive structure disposed on the lens's anterior surface provides a near-focus and an intermediate-focus optical power, and

FIGURE 11 is a schematic side view of an IOL according to a comparative example not covered by the claims having a central refractive region and an outer refractive region, which provide different refractive focusing powers.

## DETAILED DESCRIPTION

The present invention generally provides multifocal ophthalmic lenses, e.g., multifocal intraocular lenses, that employ a refractive region for providing a refractive focusing power and a diffractive region for providing two diffractive focusing powers. In many cases, the refractive focusing power provided by the lens corresponds to a far-focus optical power that is substantially equal to one of the diffractive focusing powers while the other diffractive power corresponds to a near-focus optical power. As such, in many cases, the focusing properties of the lenses are dominated by their far-focus ability, especially for small pupil sizes. In the embodiments that follow, the salient features of various aspects of the invention are discussed in connection with intraocular lenses (IOLs). The teachings of the invention can also be applied to other ophthalmic lenses, such as contact lenses. The term "intraocular lens" and its abbreviation "IOL" are used herein interchangeably to describe lenses that are implanted into the interior of the eye to either replace the eye's natural lens or to otherwise augment vision regardless of whether or not the natural lens is removed. Intracorneal lenses and phakic intraocular lenses are examples of lenses that may be implanted into the eye without removal of the natural lens.

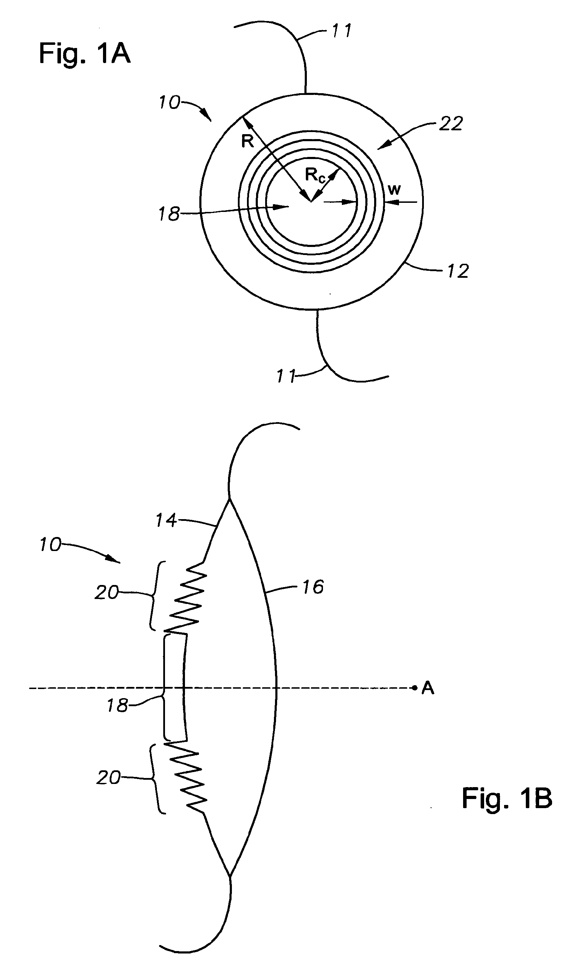

FIGURES 1A and 1B schematically depict a multifocal intraocular lens (IOL) 10 according to a comparative example not covered by the claims that includes an optic 12 having an anterior surface 14 and a posterior surface 16, which are disposed about an optical axis OA. As discussed in more detail below, the IOL 10 provides a far as well as a near focusing power. While in this example, the IOL has a bi-convex profile (each of the anterior and posterior surfaces has a convex profile), in other examples, the IOL can have any other suitable profile, e.g., convex-concave, plano-convex, etc. In some implementations, the optic 12 can have a maximum radius (R) from the optical axis OA in a range of about 2 mm to about 4 mm.

The anterior surface 14 includes a central refractive region 18, which is surrounded by an annular diffractive region 20, and an outer refractive region 22. In many implementations, the central refractive region 18 can have a radius ($R_c$) relative to the optical axis OA in a range of about 0.25 mm to about 1 mm - though other radii can also be employed. In this example, the posterior surface 16 does not include any diffractive structures, though in other embodiments it can include such structures. As discussed further below, the central refractive region 18 of the anterior surface contributes to the refractive focusing power of the optic, which corresponds in this example to the IOL's far-focus optical power. By way of example, in some cases, the optic's distance power can be in a range of about -5 to about +55 Diopters and more typically in a range of about 6 to about 34 Diopters, or in a range of about 18 to about 26 Diopters.

In this example, the base profiles of both the anterior surface 14 and the posterior surface 16 are substantially spherical with curvatures that are chosen, together with the index of refraction of the material forming the optic, such that the central refractive region of the IOL would function effectively as a monofocal refractive lens with a desired focusing power, e.g., one in the aforementioned range. In other words, for small pupil sizes, the IOL provides a single refractive focusing power.

In some other example, one or both lens surfaces can exhibit aspherical base profiles adapted to control aberrations such as by reducing the depth-of-focus (e.g., to facilitate the generation of a sharp refractive focus). By way of example, an IOL in accordance with such an example can comprising an optic having an anterior surface and a posterior surface. The anterior surface can include a refractive central region that generates, in cooperation with the posterior surface, a refractive optical power. Similar to the previous example, a diffractive region can surround the refractive central region. The diffractive region can, in turn, be surrounded by a refractive outer region. In such an example, the anterior surface has an aspheric base profile. In other words, the base profile of the anterior surface differs from a putative spherical profile. In many implementations, the asphericity of the base profile is designed to facilitate the generation of a single refractive focus by the central refractive region of the lens by controlling aberrations. For example, the aspheric base profile of the anterior surface can be characterized by a negative conic constant, which can be selected based on the refractive power of the lens, that controls aberration effects such that the central refractive portion of the lens would provide a sharp refractive focus. By way of example, the conic constant can be in a range of about -10 to about -1000 (e.g., -27). Though in this example, the base profile of the posterior surface is substantially spherical, in other examples, the base profile of the posterior surface can also exhibit a selected degree of asphericity such that the combined aspherical profiles of the two surfaces would facilitate the generation of a single refractive focus by the central portion of the lens. In other implementations, the central refractive zone can have a spherical profile in order to facilitate the generation of a single refractive focus, even when the surface has an otherwise aspheric base profile.

Referring again to FIGURES 1A and 1B, the optic 12 can be formed of any suitable biocompatible material. Some examples of such materials include, without limitation, soft acrylic, silicone, hydrogel or other biocompatible polymeric materials having a requisite index of refraction for a particular application of the lens. In many implementations, the index of refraction of the material forming the optic can be in a range of about 1.4 to about 1.6 (e.g., the optic can be formed of a lens material commonly known as Acrysof® (a cross-linked copolymer of 2-phenylethyl acrylate and 2-phenylethyl methacrylate) having an index of refraction of 1.55)

The exemplary IOL 10 also includes a plurality of fixation members (e.g., haptics) 11 that facilitate placement of the IOL in a patient's eye. The fixation members 11 can also be formed of suitable polymeric materials, such as polymethylmethacrylate, polypropylene and the like.

As noted above, the optic 12 also includes a diffractive region 20, which is disposed on its anterior surface 14, though in other examples it can be disposed on the posterior surface or on both surfaces. The diffractive region 20 forms an annular region surrounding the central refractive region 18 of the optic's anterior surface. In this exemplary embodiment, the diffractive region 20 provides a far-focus optical power as well as a near-focus power. In this example, the far-focus optical power provided by the diffractive structure is substantially similar to the refractive focusing power provided by the IOL's central refractive region. The near-focus optical power provided by the diffractive region can be, e.g., in a range of about 1 D to about 4 D, though other values can also be used. In some implementations, the diffractive region 20 can have a width (w) in a range of about 0.5 mm to about 2 mm, though other values can also be employed.

Although in some embodiments the diffractive region can extend to the outer boundary of the optic 12, in this embodiment, the diffractive region is truncated. More specifically, the diffractive region is disposed between the lens's central refractive region 18 and its outer refractive region 22. Similar to the refractive central region, the outer refractive region provides a single refractive focusing power, which in this case is substantially equal to the refractive power provided by the central region. In other words, the IOL's central and the outer refractive regions contribute only to the lens's far-focus power, while the diffractive region (herein also referred to as the zonal diffractive region) directs light energy incident thereon into both the far and near foci of the lens.

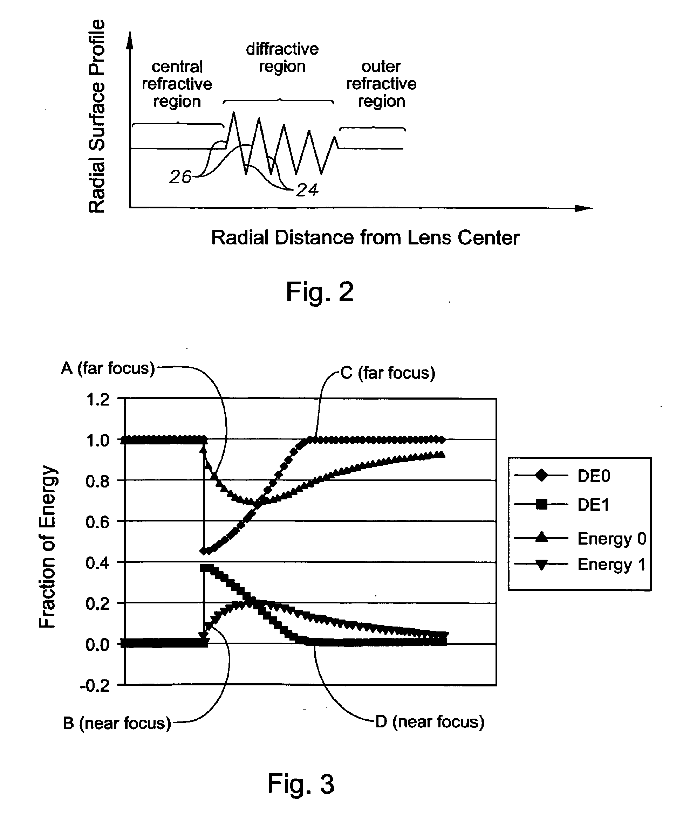

As shown schematically in FIGURE 2, which is a radial profile of the anterior surface without the base profile of the surface, in this exemplary example, the diffractive region 20 is formed of a plurality of diffractive zones 24 disposed on an underlying base curve of the anterior surface 14. The number of the diffractive zones can be in a range of about 2 to about 20, though other numbers can also be employed. The diffractive zones 24 are separated from one another by a plurality of steps 26. In this exemplary implementation, the heights of the steps 26 are non-uniform. More specifically, in this example, the step heights decrease as a function of increasing distance from a center of the anterior surface (the intersection of the optical axis OA with the anterior surface). In other words, the steps are apodized to exhibit decreasing heights as a function of increasing radial distance from the lens's optical axis. As discussed in more detail below, in other embodiments and examples, the step heights can exhibit other types of non-uniformity, or alternatively, they can be uniform. The schematic radial profile depicted in FIGURE 2 also shows that the curvatures of the IOL's central and outer refractive regions correspond to the base curvature of the anterior surface (hence these regions are shown as flat in the figure)

The steps are positioned at the radial boundaries of the diffractive zones. In this exemplary embodiment, the radial location of a zone boundary can be determined in accordance with the following relation:

$$r_i^2 = r_0^2 + 2i\lambda f \qquad \text{Equation (1),}$$

wherein

$i$ denotes the zone number
$r_0$ denotes the radius of the central refractive zone,
$\lambda$ denotes the design wavelength, and
$f$ denotes a focal length of the near focus.

In some embodiments, the design wavelength $\lambda$ is chosen to be 550 nm green light at the center of the visual response.

With continued reference to FIGURE 2, in some cases, the step height between adjacent zones, or the vertical height of each diffractive element at a zone boundary, can be defined according to the following relation:

$$\text{Step height} = \frac{\lambda}{2(n_2 - n_1)} f_{apodize} \qquad \text{Equation (2),}$$

wherein

$\lambda$ denotes the design wavelength (e.g., 550 nm),
$n_2$ denotes the refractive index of the material from which the lens is formed,
$n_1$ denotes the refractive index of a medium in which the lens is placed,
and $f_{apodize}$ represents a scaling function whose value decreases as a function of increasing radial distance from the intersection of the optical axis with the anterior surface of the lens. For example, the scaling function can be defined by the following relation:

$$f_{apodize} = 1 - \left\{ \frac{(r_i - r_{in})}{(r_{out} - r_{in})} \right\}^{exp}, r_{in} \le r_i \le r_{out} \qquad \text{Equation (3),}$$

wherein

$r_i$ denotes the radial distance of the $i^{th}$ zone,
$r_{in}$ denotes the inner boundary of the diffractive region as depicted schematically in FIGURE 2,
$r_{out}$ denotes the outer boundary of the diffractive region as depicted schematically in FIGURE 2A, and

*exp* is a value chosen based on the relative location of the apodization zone and a desired reduction in diffractive element step height. The exponent *exp* can be selected based on a desired degree of change in diffraction efficiency across the lens surface. For example, *exp* can take values in a range of about 2 to about 6.

As another example, the scaling function can be defined by the following relation:

$$f_{apodize} = 1 - (\frac{r_i}{r_{out}})^3 \qquad\qquad \text{Equation (4),}$$

wherein

$r_i$ denotes the radial distance of the i$^{th}$ zone, and
$r_{out}$ denotes the radius of the apodization zone.

Referring again to FIGURE 2, in this exemplary example, each step at a zone boundary is centered about the base profile with half of its height above the base profile and the other half below the profile. Further details regarding selection of the step heights can be found in U.S. Patent No. 5,699,142.

In use, the central refractive region provides a single far focus refractive power such that the IOL 10 effectively functions as a monofocal refractive lens for small pupil sizes, that is the pupil sizes less than or equal to the radial size of the central refractive region. For larger pupil sizes, while the central region continues to provide a single far-focus optical power, the diffractive region begins to contribute to the IOL's focusing power by providing two diffractive focusing powers: one substantially equal to the refractive far-focus power of the central region and the other corresponding to a near-focus power. As the pupil size increases further, the outer refractive region 22 also contributes - refractively - to the far-focus power of the lens. The fraction of the light energy distributed to the near focus relative to the far focus can be adjusted, e.g., via the sizes of the central and outer refractive regions as well as the parameters (e.g., step heights) associated with the diffractive region. Further, in cases in which the step heights are apodized, this fraction can change as a function of the pupil size. For example, the decrease in the step heights of the diffractive structure results in an increase in the fraction of the light energy transmitted to the far focus by the diffractive structure as the pupil size increases.

By way of further illustration, FIGURE 3 depicts graphs corresponding to local and integrated distributions of light energy emanating from a distance object that is incident on a hypothetical implementation of the IOL 10 as a function of pupil size between the far and near foci of the lens (graphs A and B correspond to integrated distribution and graphs C and D correspond to local distribution). For small pupil sizes (e.g., less than about 1.5 mm in this example), all of the light incident on the IOL (ignoring scattering and other losses) is directed to the IOL's far focus by the IOL's central refractive region. For intermediate pupil sizes (e.g., for pupil diameters in a range of about 1.5 mm to about 4.5 mm in this example), some of the light is directed to the IOL's near focus, which is provided by the IOL's diffractive structure, while the remaining light is directed to the IOL's far-focus. As the pupil size traverses the diffractive region, the local fraction of the energy directed to the near focus decreases due to the apodization of the step heights separating different zones of the diffractive region. For large pupil sizes, the IOL's outer refractive region begins to contribute to the IOL's far-focus optical power.

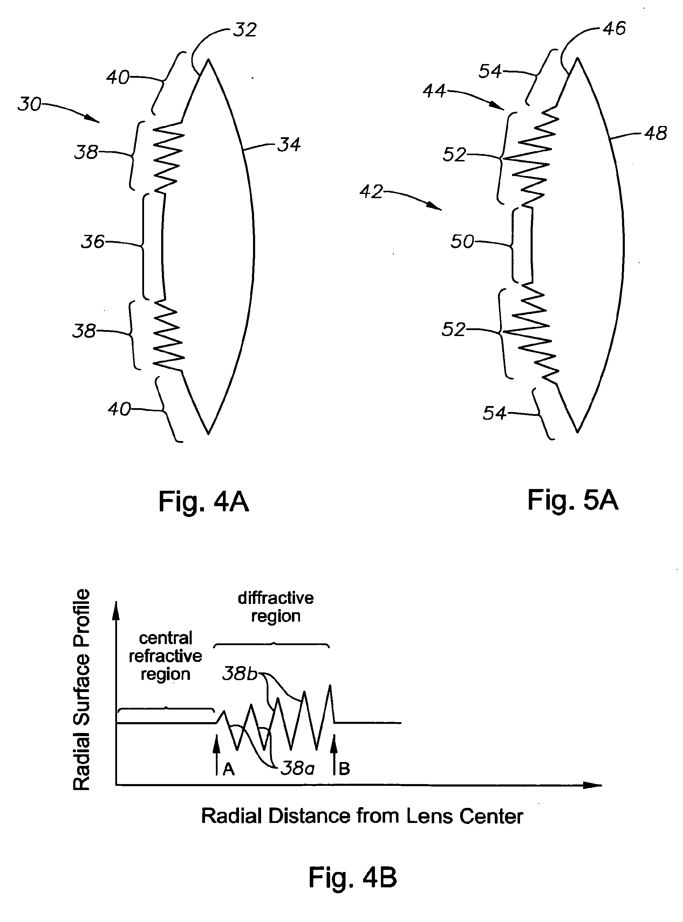

The apodization of the diffractive region is not limited to the one discussed above. In fact, a variety of apodization of the step heights can be employed. By way of example, with reference to FIGURE 4A and 4B, in some embodiments, an IOL 30 can include an anterior surface 32 and a posterior surface 34, where the anterior surface is characterized by a central refractive region 36, an annular diffractive region 38 that surrounds the central refractive region 34, and an outer refractive region 40. The annular diffractive region is formed by a plurality of diffractive zones 38a that are separated from one another by a plurality of steps 38b, where the steps exhibit increasing heights from an inner boundary A of the diffractive region to an outer boundary B thereof.

Such an apodization of the step heights is herein referred to as "reverse apodization." Similar to the previous embodiment, the diffractive region contributes not only to the IOL's far-focus optical power but also to its near-focus power, e.g., the near-focus power can be in a range of about 1 to about 4 D. However, unlike the previous embodiment, the percentage of the incident light energy transmitted by the diffractive region to the far focus decreases as the pupil size increases (due to the increase in the step heights as a function of increasing radial distance from the optical axis).

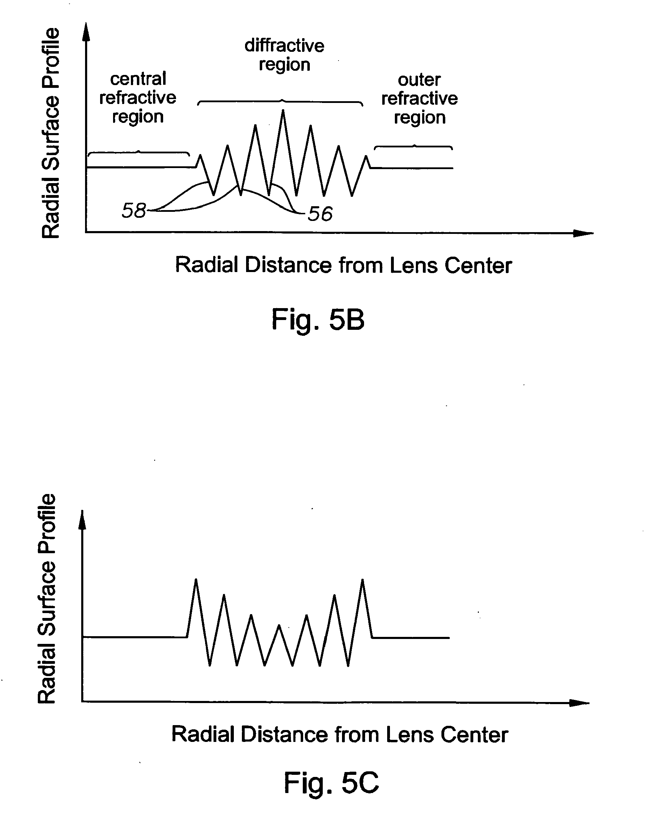

In other embodiments, the step heights in the diffractive region can increase from the region's inner boundary to reach a maximum value at an intermediate location within that region followed by a decrease to the region's outer boundary. By way of example, FIGURE 5A depicts such an IOL 42 having an optic 44 characterized by an anterior surface 46 and a posterior surface 48. Similar to the previous embodiments, the anterior surface 46 includes a central refractive region 50, an annular diffractive region 52 that surrounds the refractive region, and an outer refractive region 54 that in turn surrounds the diffractive region. With reference to the radial profile of the anterior surface presented in FIGURE 5B, the annular diffractive region includes a plurality of diffractive zones 56 separated from another by a plurality of steps 58, where the step heights exhibit an increase followed by a decrease as a function of increasing radial distance from the center of the lens. Alternatively, in another embodiment shown schematically in FIGURE 5C, the step heights show a decrease followed by an increase as a function of increasing distance from the lens center.

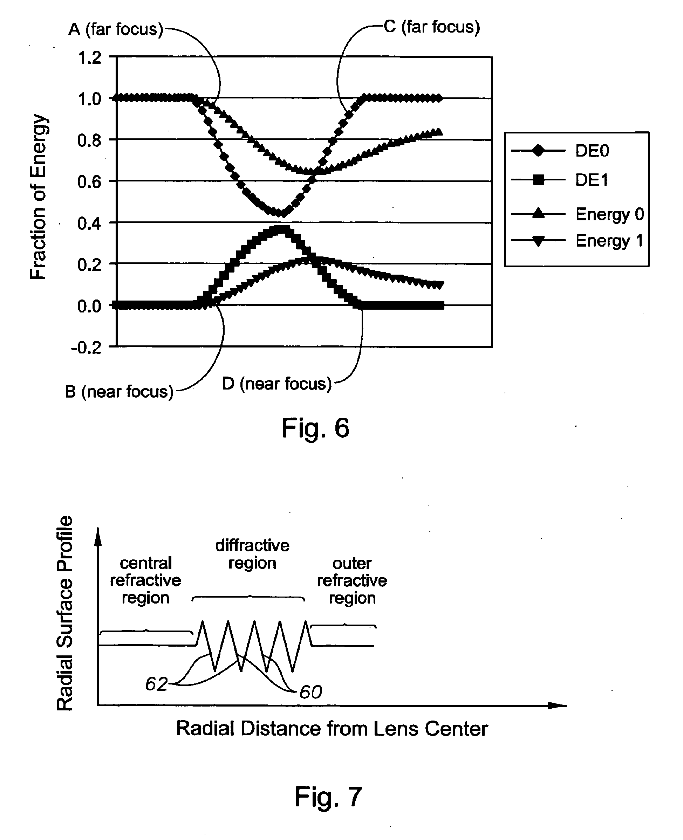

By way of illustration, FIGURE 6 schematically depicts the distribution of light energy from a distant object incident on a hypothetical implementation of the IOL 42, which has a diffractive region characterized by an initial increase followed by a decrease of step heights, as a function of pupil size. The IOL is assumed to have a diameter of 6 mm with each of the central refractive region, the diffractive region and the outer refractive region having a radial extent of 1.5 mm. The graphs A and B show, respectively, an integrated distribution of energy into near and far foci and the graphs C and D show, respectively, local distribution of energy into those foci. For small pupil sizes (e.g., pupil diameters less than the radial extent of the IOL's central refractive region), the IOL effectively functions as a monofocal refractive lens by refractively focusing the light incident thereon onto the IOL's far focus. As the pupil size increases to expose the IOL's diffractive region to some of the incident light, a fraction of the incident light is diverted to the IOL's near focus. Due to the non-uniformity of the step heights, the local fraction increases to reach a maximum followed by a decrease towards to boundary of the diffractive structure with the outer refractive region (the local fraction of light directed to the far focus, in turn, decreases to reach a minimum followed by an increase towards the boundary of the diffractive structure with the outer refractive region). As the pupil size increases even further, the outer refractive region begins to refractively contribute to the IOL's far-focus power.

According to a comparative example not covered by the claims, the step heights separating different zones of the diffractive region can be substantially uniform (e.g., within manufacturing tolerances). By way of illustration, FIGURE 7 schematically depicts a radial profile of a surface of such a lens (e.g., the anterior surface of the lens) from which the underlying base profile has been subtracted. The radial surface profile indicates that the surface includes a central refractive region A (with a curvature that is substantially equal to the base curvature of the surface), a diffractive region B and an outer refractive region C. The diffractive region B is characterized by a plurality of diffractive zones 60 that are separated from one another by a plurality of steps 62. The heights of the steps 62 are substantially uniform.

By way of example, in some implementations of an IOL having a substantially uniform step height, which provides a selected phase shift at each zone boundary, the radial location of a zone boundary can be determined in accordance with the following relation:

$$r_i^2 = r_0^2 + 2i\lambda f \qquad \text{Eq. (5)}$$

wherein

  $i$ denotes the zone number ($i = 0$ denotes the central zone)
  $\lambda$ denotes the design wavelength,
  $f$ denotes a focal length of the near focus, and
  $r_0$ denotes the radius of the central zone

In some embodiments, the design wavelength $\lambda$ is chosen to be 550 nm green light at the center of visual response. In some cases, the radius of the central zone ($r_0$) can be set to be $\sqrt{\lambda f}$ .

Further, the step height between adjacent zones can be defined in accordance with the following relation:

$$\text{Step height} = \frac{b\lambda}{(n_2 - n_1)} \qquad \text{Eq. (6)}$$

wherein

  $\lambda$ denotes the design wavelength (e.g., 550 nm),
  $n_2$ denotes the refractive index of the material from which the lens is formed,
  $n_1$ denotes the refractive index of the medium in which the lens is placed, and

*b* is a fraction, e.g., 0.5 or 0.7.

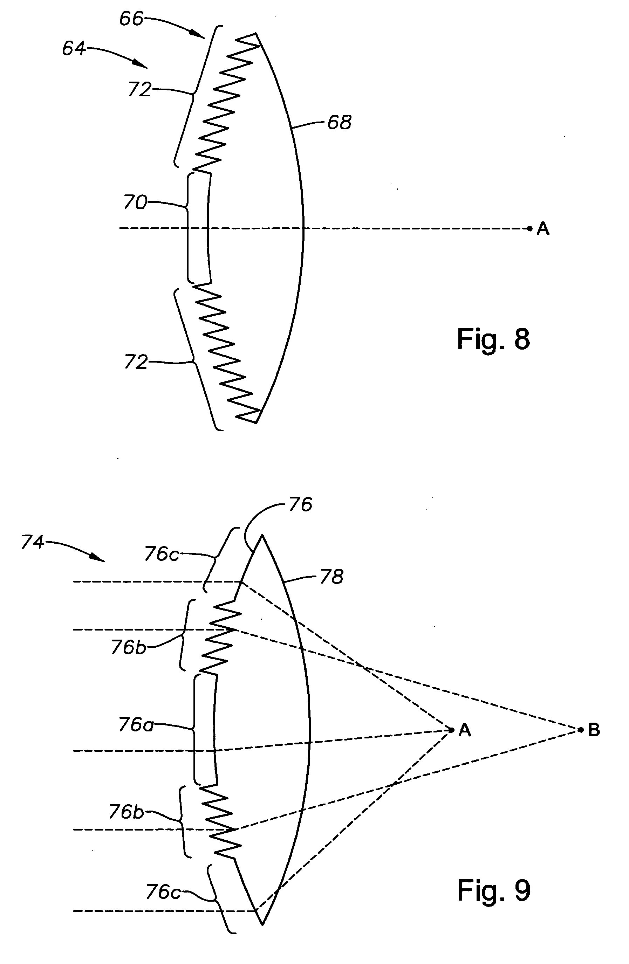

In some embodiments, the diffractive region can extend from the outer boundary of the central refractive region to the outer boundary of the optic. By way of example, FIGURE 8 schematically depicts such an IOL 64 that includes an anterior surface 66 and a posterior surface 68. The anterior surface includes a central refractive region 70 that, in cooperation with the refractive posterior surface, imparts to the optic a refractive far-focus power. A diffractive region 72 disposed on the anterior surface extends from the outer boundary of the central refractive region to the outer boundary of the optic, and provides a diffractive near-focus and a diffractive far-focus optical power. In this exemplary implementation, the diffractive far-focus power is substantially equal to the refractive far-focus power provided by the optic's refractive central region. Although in this example the diffractive region is formed by a plurality of diffractive zones separated by steps having substantially uniform heights, in other implementations the step heights can be non-uniform (e.g., they can be apodized).

Although in the above embodiments the central region provides a far-focus optical power, in other comparative examples, the central region can provide a near-focus optical power with the diffractive region providing the optic's far-focus optical power while also contributing - diffractively - to its near-focus optical power. By way of example, with reference to FIGURE 9, such an IOL 74 can include an anterior surface 76 and a posterior surface 78, where the anterior surface includes a central refractive region 76a, an annular diffractive region 76b, and an outer refractive region 76c. In this embodiment, the base curvatures of the anterior and the posterior surfaces, together with the index of refraction of the material forming the lens, are selected such that the posterior surface and the central region of the anterior surface cooperatively provide a near-focus optical power, e.g., a focusing power in a range of about 1 to about 4 D. Such a near-focus is schematically shown as focus A in FIGURE 9. Similarly, the outer refractive region, in combination with the posterior surface, also contributes to the optic's near-focus optical power. The diffractive region, in turn, provides a far-focus optical power (corresponding to focus B shown in the figure) as well as a near-focus optical power, where the diffractive and the refractive near-focus powers are substantially equal. In this example, the diffractive region includes a plurality of diffractive zones that are separated from one another by a plurality of steps having substantially uniform step heights. In other implementations, the step heights can be non-uniform, e.g., in a manner discussed above in connection with the previous embodiments.

The fraction of the light energy directed by the diffractive region of the IOL 74 into the near and far foci can be adjusted, e.g., based on the needs of the patient. By way of example, in many implementations of the IOL 74, the diffractive region is adapted to direct most of the light energy into the far-focus, as the refractive portions of the lens direct the light incident thereon to the IOL's near focus. By way of example, the ratio of the light energy directed to the far focus relative to that directed to near focus by the diffractive region can be in range of about 2 to about 4. A pending U.S. patent application entitled "Truncated Diffractive Intraocular Lenses" having a serial number 11/444,112 filed on August 23, 2006, which is herein incorporated by reference in its entirety, discloses various ways of adjusting a diffractive structure to shift the ratio of the light energy directed to the near and far-foci of a multifocal ophthalmic lens.

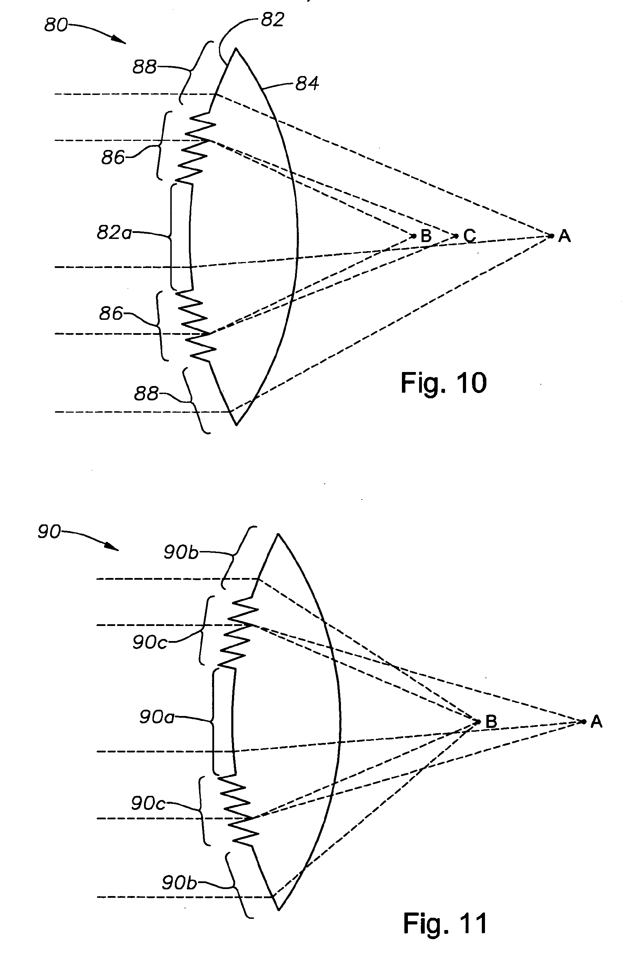

In some other comparative examples, the IOL's central refractive region can provide a far-focus refractive power while the diffractive region provides a near-focus and an intermediate-focus power. By way of illustration, FIGURE 10 schematically depicts such a lens 80 having a central refractive region, characterized by a central refractive region 82a of an anterior surface 82 of the lens and a refractive posterior surface 84 thereof, surrounded by a diffractive region 86. Although in this example the heights of the steps separating different diffractive zones of the diffractive region are substantially uniform, in other implementations the step heights can be non-uniform. An outer refractive region 88, in turn, surrounds the diffractive region 86. As shown schematically in FIGURE 10, the optic's central and outer refractive regions provide a far focus A while the diffractive region is adapted to generate a near focus B as well as an intermediate focus C. In some implementations, the focusing power associated with the far focus can be in a range of about 6 to about +34 D while the power associated with the near focus can be in a range of about 1 to about 4 D with the power corresponding to the intermediate focus lying in a range of about 0.5 D to about 3 D.

In some other embodiments, an IOL can include a central refractive region, an annular diffractive region disposed on a surface thereof, and an outer refractive region, where the central and the outer refractive regions provide different refractive focusing powers. By way of example, as shown schematically in FIGURE 11, a central refractive region 90a of such an IOL 90 can contribute to the IOL's far-focus optical power (corresponding to far focus A) while an outer refractive region 90b of the IOL contributes - refractively - to the IOL's near-focus optical power (corresponding to near focus B). A diffractive region 90c, in turn, contributes -diffractively - to both the near and the far focusing powers of the IOL. Such a difference in the refractive focusing properties of the central and outer regions can be achieved, e.g., by configuring the outer region of one or both of the lens surfaces to have a different surface curvature (surface profile) than that of the respective central region.

In some cases, the base profile of at least one of the lens surfaces can exhibit a selected degree of asphericity to control aberrations, such as to control depth-of-focus. For example, the anterior surface on which a diffractive region is disposed can exhibit a spherical profile while the posterior surface exhibits a certain degree of asphericity. By way of example, further teachings regarding configuring one or more of the lens surfaces to have aspherical profiles can be found in pending U.S. patent application entitled "Intraocular Lens" having a publication number US 2006/244904, filed on April 4, 2006.

In other cases, at least one of the lens surfaces can have a toric base profile (a profile characterized by two different curvatures along two orthogonal directions of the surface) to help correct astigmatism.

In some embodiments, the biocompatible polymeric material of the optic can be impregnated one or more dyes such that the lens can provide some degree of filtering of blue light. Some examples of such dyes are provided in U.S. Patent Nos. 5,528,322 (entitled "Polymerizable Yellow Dyes And Their Use In Ophthalmic Lenses"), 5,470,932 (entitled "Polymerizable Yellow Dyes And Their Use In Ophthalmic Lenses"), 5,543,504 (entitled "Polymerizable Yellow Dyes And Their Use In Ophthalmic Lenses), and 5,662,707 (entitled "Polymerizable Yellow Dyes And Their Use In Ophthalmic Lenses).

A variety of known manufacturing techniques can be employed to form a ophthalmic lens (e.g., an IOL) in accordance with the teachings of the invention. For example, such techniques can be employed to initially form a refractive optic and subsequently generate an annular diffractive region on one of the surfaces of the optic such that the diffractive region would surround an central refractive region of the surface.

Those having ordinary skill in the art will appreciate the certain modifications can be made to the above embodiments without departing from the scope of the invention.

**Claims**

1.  An intraocular lens (10,30,42,64,74,80,90) comprising;
    an optic (12) having an anterior surface (14) and a posterior surface (16), each of said surfaces having a central refractive region (18) for providing one refractive focusing power to the optic, and
    an annular diffractive region (20) disposed on one of said surfaces so as to at least partially surround the central refractive region of that surface to provide a near and a far diffractive focusing powers,
    **characterized in that** the central refractive regions (18) of the anterior surface (14) and the posterior surface (16) of the optic have a substantially spherical profile and are monofocal and adapted to provide a single far focus refractive power, and
    wherein the lens size is selected so that at a first pupil size the lens is a monofocal lens having a focusing power corresponding to the refractive far-focus power provided by the central refractive regions and as pupil size increases the lens is a multifocal lens with the annular diffractive region directing varying amounts of light energy to the near and far diffractive focus powers, wherein said annular diffractive region (20) comprises a plurality of diffractive zones (24) separated from one another by a plurality of steps wherein said step (3 8b) heights increase as a function of increasing distance from a center of said optic.

2.  The intraocular lens of claim 1, wherein said central refractive region (18) of any of the anterior and the posterior surface has a diameter in a range of about 0.5 mm to about 2 mm.

3.  The intraocular lens of claim 2, wherein said diffractive region outside of said central refractive region has a substantially aspheric base profile.

4.  The intraocular lens of claim 1, wherein said optic comprises an outer refractive region (22).

5.  The intraocular lens of claim 1, wherein said outer refractive region provides a focusing power substantially equal to the refractive focusing power provided by the central region.

6.  The intraocular lens of claim 1, wherein at least one of said surfaces exhibits an aspheric base profile adapted to control aberrations of the lens.

7.  The intraocular lens of claim 6, wherein said aspheric base profile is **characterized by** a negative conic constant.

**Patentansprüche**

1.  Intraokularlinse (10, 30, 42, 64, 74, 80, 90) umfassend:

    eine Optik (12) mit einer vorderen Fläche (14) und einer hinteren Fläche (16), wobei jede der Flächen einen zentralen refraktiven Bereich (18) zur Schaffung einer refraktiven Fokussierung der Optik aufweist, und einen ringförmigen diffraktiven Bereich (20), der auf einer der Flächen so vorgesehen ist, dass er den zentralen refraktiven Bereich dieser Fläche zumindest teilweise umschließt, um eine nahe und eine weite diffraktive Fokussierung zu schaffen,

**dadurch gekennzeichnet, dass** die zentralen refraktiven Bereiche (18) der vorderen Fläche (14) und der hinteren Fläche (16) der Optik ein im wesentlichen kugelförmiges Profil aufweisen und monofokal sind und für eine einzige Brechkraft mit langer Brennweite ausgelegt sind, und

wobei die Linsengröße dermaßen gewählt ist, dass bei einer ersten Pupillengröße die Linse eine Monofokallinse ist, die eine Fokussierung entsprechend dem refraktiven Fern-Fokus der zentralen refraktiven Bereiche aufweist, und mit zunehmender Pupillengröße die Linse eine Multifokallinse mit dem ringförmigen diffraktiven Bereich ist, womit unterschiedliche Mengen von Lichtenergie zur nahen und fernen diffraktiven Fokussierung gelenkt werden,

wobei der ringförmige diffraktive Bereich (20) eine Vielzahl von diffraktiven Zonen (24) umfasst, welche voneinander durch eine Vielzahl von Stufen getrennt sind, wobei die Stufenhöhen (38b) als Funktion des zunehmenden Abstands von einem Zentrum der Optik zunehmen.

2. Intraokularlinse nach Anspruch 1, wobei der zentrale refraktive Bereich (18) von jeder der vorderen und der hinteren Fläche einen Durchmesser in einem Bereich von etwa 0,5 mm bis etwa 2 mm hat.

3. Intraokularlinse nach Anspruch 2, wobei der diffraktive Bereich außerhalb des zentralen refraktiven Bereichs ein im Wesentlichen asphärisches Basisprofil aufweist.

4. Intraokularlinse nach Anspruch 1, wobei die Optik einen äußeren refraktiven Bereich (22) aufweist.

5. Intraokularlinse nach Anspruch 1, wobei der äußere refraktive Bereich eine Fokussierung zeigt, die im Wesentlichen gleich der refraktiven Fokussierung des zentralen Bereichs ist.

6. Intraokularlinse nach Anspruch 1, wobei mindestens eine der genannten Flächen ein asphärisches Basisprofil aufweist, welches dazu geeignet ist, die Aberrationen der Linse zu steuern.

7. Intraokularlinse nach Anspruch 6, wobei sich das asphärische Basisprofil durch eine negative konische Konstante auszeichnet.

## Revendications

1. Lentille intraoculaire (10, 30, 42, 64, 74, 80, 90) comprenant :

une optique (12) ayant une surface antérieure (14) et une surface postérieure (16), chacune desdites surfaces ayant une région réfractive centrale (18) pour procurer une puissance de focalisation réfractive à l'optique, et une région diffractive annulaire (20) disposée sur l'une desdites surfaces de manière à au moins partiellement entourer la région réfractive centrale de cette surface afin de procurer une puissance de focalisation diffractive proche et une puissance de focalisation diffractive éloignée,

**caractérisée en ce que** les régions réfractives centrales (18) de la surface antérieure (14) et de la surface postérieure (16) de l'optique ont un profil sensiblement sphérique et sont monofocales et adaptées pour procurer une puissance réfractive de focalisation éloignée unique, et

dans laquelle la taille de la lentille est sélectionnée de manière à ce que, à une première taille de pupille, la lentille est une lentille monofocale ayant une puissance de focalisation correspondant à la puissance de focalisation réfractive éloignée procurée par les régions réfractives centrales et, lorsque la taille de la pupille augmente, la lentille est une lentille multifocale avec la région diffractive annulaire dirigeant diverses quantités d'énergie lumineuse vers les puissances de focalisation diffractives proche et éloignée, dans laquelle ladite région diffractive annulaire (20) comprend une pluralité de zones diffractives (24) séparées les unes des autres par une pluralité d'incréments, où lesdites hauteurs d'incrément (38b), fonctions de l'accroissement de la distance au centre de ladite optique, augmentent.

2. Lentille intraoculaire selon la revendication 1, dans laquelle ladite région réfractive centrale (18) de l'une quelconque de la surface antérieure et de la surface postérieure a un diamètre situé dans une plage s'étendant d'environ 0,5 mm à environ 2 mm.

**3.** Lentille intraoculaire selon la revendication 2, dans laquelle ladite région réfractive extérieure à ladite région diffractive centrale a un profil de base sensiblement asphérique.

**4.** Lentille intraoculaire selon la revendication 1, dans laquelle ladite optique comprend une région réfractive extérieure (22).

**5.** Lentille intraoculaire selon la revendication 1, dans laquelle ladite région réfractive extérieure procure une puissance de focalisation sensiblement égale à la puissance de focalisation réfractive procurée par la région centrale.

**6.** Lentille intraoculaire selon la revendication 1, dans laquelle au moins une desdites surfaces présente un profil de base asphérique adapté pour contrôler les aberrations de la lentille.

**7.** Lentille intraoculaire selon la revendication 6, dans laquelle ledit profil de base asphérique est **caractérisé par** une constante conique négative.

Fig. 1A

Fig. 1B

**Fig. 2**

**Fig. 3**

Fig. 4A

Fig. 5A

Fig. 4B

14

**Radial Surface Profile**

central refractive region

diffractive region

outer refractive region

58    56

Radial Distance from Lens Center

## Fig. 5B

**Radial Surface Profile**

Radial Distance from Lens Center

## Fig. 5C

Fig. 6

Fig. 7

66
64
72
68
70
A

Fig. 8

76
74
76c
78
76b
76a
A
B
76b
76c

Fig. 9

80

88

82

84

86

82a

86

88

B C A

**Fig. 10**

90

90b

90c

90a

90c

90b

B A

**Fig. 11**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7073906 A
- US 5699142 A
- US 11444112 B
- US 2006244904 A
- US 5528322 A
- US 5470932 A
- US 5543504 A
- US 5662707 A